Europäisches Patentamt

European Patent Office

Office européen des brevets

(11) Publication number: 0 385 445
A2

# EUROPEAN PATENT APPLICATION

(21) Application number: 90103915.6

(51) Int. Cl.⁵: A61K 9/107, A61K 9/50

(22) Date of filing: 28.02.90

(30) Priority: 28.02.89 JP 47591/89
31.08.89 JP 224842/89

(43) Date of publication of application:
05.09.90 Bulletin 90/36

(84) Designated Contracting States:
AT BE CH DE DK ES FR GB GR IT LI NL SE

(71) Applicant: Horikoshi, Isamu
4808, Yoshizukuri
Toyama-shi, Toyama-ken(JP)

(72) Inventor: Horikoshi, Isamu
4808, Yoshizukuri
Toyama-shi, Toyama-ken(JP)
Inventor: Muranishi, Shozo, 562-19,
Kansantachibana-cho
Ichijyo Agaru, Nishiiru, Karasumadohri
Kamigyo-ku, Kyoto-shi, Kyoto(JP)
Inventor: Watanabe, Jun
6-11-2, Ishiodai
Kasugai-shi, Aichi-ken(JP)
Inventor: Yamauchi, Kunio
2-2-33, Matsunami
Chigasaki-shi, Kanagawa-ken(JP)
Inventor: Kanno, Choemon
1023-2, Shimohirademachi
Utsunomiya-shi, Tochigi-ken(JP)
Inventor: Honda, Takashi
3742-11, Yoshizukuri
Toyama-shi, Toyama-ken(JP)

(74) Representative: Vossius & Partner
Siebertstrasse 4 P.O. Box 86 07 67
D-8000 München 86(DE)

(54) Method of forming a suspension and composition formed by said method.

(57) A method by which lipid-soluble materials that are insoluble or slightly soluble in water but which are soluble in organic solvents or materials that are slightly soluble in both water and organic solvents but the partition coefficients of which are much more favorable to organic solvents than water are suspended using fatty globule membranes in mammalian milk as suspending agents. In place of fatty globule membranes in mammalian milk, dairy products such as buttermilk and cream which contain milk fatty globule membranes may be used as suspending agents.

# *F l g. 1*

SOLUBILIZING ABILITY

▼:HCO60

■:BL9

●:BUFFER SOLUTION

▲:MFGM

1a

# METHOD OF FORMING A SUSPENSION AND COMPOSITION FORMED BY SAID METHOD

The present invention relates to a novel method of forming suspensions using fatty globule membranes in milk secreted from mammals. It also relates to a composition containing a material that is obtained by this method. More particularly, the present invention relates to:

(1) a novel method of forming suspensions of lipid-soluble materials that are slightly soluble or insoluble in water but which are soluble in organic solvents, as well as materials that are slightly soluble in both water and organic solvents but the partition coefficients of which are much more favorable to organic solvents than water (these two types of materials are hereinafter collectively referred to as "lipid-soluble materials"), in particular, lipid-soluble drugs and those drugs such as certain steroids that are slightly soluble in both water and organic solvents but the partition coefficients of which are much more favorable to organic solvents than water (these two types of drugs are hereinafter collectively referred to as "lipid-soluble drugs"); and

(2) a novel method of formulating physiologically active peptides into a dosage form suitable for oral administration.

It has long been known from experience that considerable difficulty is involved in administering water-insoluble or slightly soluble compounds orally and having them exhibit their pharmaceutical efficacy with high reproducibility.

However, remarkable advances have been made in the study of oral administration of lipid-soluble compounds that are slightly soluble or insoluble in water, particularly with respect to (1) absorption by digestive tracts, (2) distribution among various organs either by blood circulation or via lymph fluids, (3) metabolism in organs and (4) excretion of those compounds or metabolites thereof into urine or bile. Very active efforts are also being made to develop "drug delivery systems" by which drugs can be locally concentrated on specific organs or cancer tissues.

With the recent advances in pharmacology, significant improvements have been achieved in the techniques of evaluating drug efficacy and it has become possible to perform very precise measurements of the pharmaceutical efficacy of lipid-soluble compounds that the slightly soluble or insoluble in water. A problem with the development of lipid-soluble, pharmacologically active substances that can be used as medicines is that they have to be formulated in dosage forms that allow them to exhibit the intended pharmaceutical efficacy with high reproducibility. As already mentioned, it has long been known that considerably great difficulty is involved in administering water-insoluble or slightly soluble compounds orally and having them exhibit the intended pharmaceutical efficacy with high reproducibility. Under these circumstances, intensive studies have been conducted to determine the reason why orally administered lipid-soluble drugs were incapable of exhibiting their efficacy with high reproducibility and several observations that are unique to lipid-soluble drugs (i.e. not found with water-soluble compounds) have been obtained concerning such aspects as "absorption by digestive tracts", "distribution among organs", "excretion" and "metabolism". The facts that have become clear with respect to the absorption, excretion, distribution among organs and metabolism of lipid-soluble drugs as a result of the past studies may be summarized as follows.

(1) When lipid-soluble drugs that are water-insoluble or slightly soluble in water are administered orally, the rate of their absorption by digestive tracts is proportional to the surface area of drug particles. In other words, one of the factors that govern the absorption of lipid-soluble drugs by digestive tracts is the surface area of the drug particles and given the same amount of lipid-soluble drugs administered, the larger the surface area of the drug particles (i.e., the greater the diameter of these particles), the faster the rate of their absorption by digestive tracts.

(2) Lipid-soluble drugs are absorbed by passive diffusion through the mucosa of the small intestines. No matter how slightly soluble in water, lipid-soluble drugs are absorbed into the body only after a very small amount of mono-molecules dissolves in water. In other words, almost all lipid-soluble drugs are taken into the body by absorption through digestive tracts at a slower rate than water-soluble drugs but the process of absorption through the mucosa of small intestines is not a rate-limiting step. Lipid-soluble drugs dissolve in very small amounts in water but the surface area of the mucosa of small intestines is so large that the process of passive diffusion will not become a rate-limiting step.

(3) Even if drugs have a very low level of solubility in water, the rate of their absorption by digestive tracts will increase as the rate at which monomolecules are released from the drug particles into water increases. In other words, another factor that governs the absorption of lipid-soluble drugs by digestive tracts is not the level of their water solubility but the rate at which the drugs dissolve in water.

(4) When lipid-soluble drugs are absorbed through digestive tracts, they are taken into the body via two routes. One route is by passage of the drug through the portal vein and the liver to flow into the systemic circulation, whereby it is distributed among various parts of the body. The other route is by absorption into the lymph vessels in the intestines, followed by flowing into the artery through lymph in the thoracic duct. When the drugs are absorbed by the second route, they,often appear in the blood stream in their initial form. If drugs are absorbed by passage through the portal vein and the liver, their molecules are often subjected to chemical modification by the "first pass effect". Depending on dosage form, lipid-soluble drugs that are slightly soluble or insoluble in water, experience great variations in absorption ratio and absorption rate following oral administration, ranging from negligible absorption to substantially quantitative absorption.

Two different approaches have heretofore been taken to increase the absorption rate, and hence the absorption ratio, of lipid-soluble drugs. One is by pulverizing the drugs. It has been impossible to reduce the average size of drug particles to less than 150 - 200 μm by conventional comminution techniques, but today the introduction of new equipment and methods has made it fairly easy to pulverize drug particles to an average diameter of 2 μm in commercial operations. The advances in the technology of suspending drugs in water and formulating them in a suitable dosage form have also been remarkable and techniques have been developed by which oil droplets of several microns in diameter can be stored as a stable suspension at room temperature for a prolonged period. Another method that has been commercialized is to mix lipid-soluble drugs with surfactants to form transparent micelles that can be absorbed by digestive tracts at increased rate.

The second approach for increasing the absorption rate, and hence the absorption ratio, of lipid-soluble drugs has been directed to enhancement of the rate at which the molecules of a drug contained in particles or oil globules are released into water. It is well known that the dissolution rate and absorption ratio of an antibiotic, Novobiocin (trade name), differs greatly depending on its crystal form. Studies on the rate of dissolution of the molecules of lipid-soluble drugs into water have revealed that compared to crystalline form, drugs in the form of an amorphous powder or solid solution are dissolved at a faster rate, thereby permitting the drug to be absorbed at a faster rate and in a greater amount.

In spite of the recent remarkable advances in the technology of formulating drugs in dosage forms, no satisfactory improvements have been seen in lipid-soluble drugs in terms of the reproducibility and efficiency of absorption when they are administered into the body perorally, particularly with respect to the rate and quantity of their absorption.

The administration of physiologically active peptides has heretofore been limited to parenteral routes, namely, by injection. This is because most of the physiologically active peptides have high molecular weights and it is contrary to common sense that such high-molecular weight substances, when administered perorally, should be absorbed in large amounts by the intestinal tract. Further, orally administered physiologically active peptides are readily hydrolyzed by proteolytic enzymes secreted in the digestive tract, so they will have been hydrolyzed and consequently their amount will have decreased considerably by the time when they reach the site of absorption. For these reasons, physiologically active peptides are conventionally administered as injections but when physiologically active foreign peptides are administered in the form of injections, antibodies are produced in response to non-autoantigens from dissimilar proteins, with the potential of causing an antigen-antibody reaction shock. To eliminate this problem, many attempts have been made to formulate physiologically active peptides in dosage forms that are suitable for oral administration and the approaches that have been taken in these attempts can be roughly divided into the following three types. In one approach, a protease inhibitor is administered simultaneously with a physiologically active peptide so that the latter can be absorbed by the intestinal tract with maximum protection insured against hydrolysis that may occur within the digestive tract. In the second approach, a physiologically active peptide is administered together with a compound that promotes its absorption by the intestinal tract, so as to insure that said peptide is taken into the body at an increased ratio. According to the third approach, a surfactant is administered simultaneously with a physiologically active peptide so that temporary damage is caused to cells in the mucosa of the intestines to enhance the permeability of said peptide through cellular membranes. In some attempts, combinations of these approaches have been adopted.

However, these approaches had the following common defects. First, only very small amounts of physiologically active peptides can be absorbed with their physiological actions retained completely intact. Second, the absorption of physiologically active peptides often lacks reproducibility. Third, the absorption of physiologically active peptides by the intestinal tract varies so greatly between individuals that the same amount of a physiologically active peptide is little absorbed by one individual whereas in another individual, it is absorbed excessively with the potential of causing a coma.

The present inventors conducted for many years a study on fatty globules present in mammalian milk.

EP 0 385 445 A2

As a result, they found that fatty globule membranes obtained by removing fat from fatty globules were suitable for use as materials with which water-insoluble or slightly water-soluble lipid-soluble drugs and physiologically active peptides could be formulated in dosage forms. The present invention has been accomplished on the basis of this finding.

The present invention relates to:

a novel method of forming a suspension using fatty globule membranes in mammalian milk as a suspending agent;

a novel method of forming a suspension of a lipid-soluble material that is slightly soluble or insoluble in water and that is soluble in organic solvents, which method is characterized by coating said lipid-soluble material with fatty globule membranes in mammalian milk;

a novel method of forming a fine aqueous suspension of a material that is slightly soluble in both water and an organic solvent but the partition coefficient of which is more favorable to the organic solvent than water, which method is characterized by coating said material with fatty globule membranes in mammalian milk;

a novel method of forming a fine aqueous suspension of a lipid-soluble material that is slightly soluble or insoluble in water and that is soluble in organic solvents, which method is characterized in that fatty globule membranes in mammalian milk and said lipid-soluble material are emulsified in water by mechanical or physical means;

a novel method of forming a fine aqueous suspension of a material that is slightly soluble in both water and an organic solvent but the partition coefficient of which is more favorable to the organic solvent than water, which method is characterized in that fatty globule membranes in mammalian milk and said material are emulsified in water by mechanical or physical means;

a novel method of forming a fine aqueous suspension of a physiologically active peptide by emulsifying said peptide with the aid of fatty globule membranes in mammalian milk;

a novel method of forming a suspension of a physiologically active peptide, which method comprises dissolving or suspending said peptide in water, adding a neutral fat and fatty globule membranes in mammalian milk, and emulsifying the mixture by mechanical or physical means;

a novel method of forming a suspension of a physiologically active peptide, which method comprises dissolving or suspending said peptide in water, adding a neutral fat, fatty globule membranes in mammalian milk and a protease inhibitor, and emulsifying the mixture by mechanical or physical means;

any one of the novel methods described above, which is further characterized in that said fatty globule membranes in mammalian milk are purified fatty globule membranes in cow milk;

any one of the novel methods described above, which is further characterized by replacing said fatty globule membranes in mammalian milk with a dairy product containing cow milk fatty globule membranes;

a novel method as described directly above which is further characterized in that the dairy product containing cow milk fatty globule membranes is buttermilk or cream;

a process for producing a pharmaceutical preparation suitable for oral administration, which process is characterized in that a suspended formulation prepared by any one of the methods described above is either directly, or after addition of an extender, spray-dried or freeze-dried to form a powder; and

a composition prepared by any one of the methods described above.

Fig. 1 is a graph showing the solubilizing ability of MFGM (milk fatty globule membrane, or fatty globule membrane in mammalian milk) to be used in the present invention;

Fig. 2 is a graph showing the emulsion stability of lecithin, Tween®80, HCO 60 and MFGM;

Fig. 3 is a graph showing the emulsion activity of various surfactants in emulsions;

Fig. 4 is a graph showing the dependency of emulsion activity of MFGM on concentration;

Fig. 5 is a graph showing the effect of sucrose aliphatic acid esters (S-1170 and P-1670) and MFGM on emulsion activity;

Fig. 6 is a graph showing the dependency of emulsion activity on the type of fats and oils;

Fig. 7 is a graph showing the concentration profile of vitamin A in lymph after administration of a vitamin A containing emulsion into the small intestine of biliary fistula rats;

Fig. 8 is a graph showing the concentration profile of vitamin A in lymph after administration of a vitamin A containing emulsion into the small intestine of intact rats;

Fig. 9 is a graph showing the concentration profile of vitamin A in lymph as observed when emulsions containing vitamin A in various oils and fats were administered into the small intestine of intact rats;

Fig. 10 is a graph showing the concentration profile of tritium labelled hydrocortisone in blood after its administration (1.5 mg/kg) to rats;

Fig. 11 is a graph showing how the blood sugar level of diabetic rats dropped after they were administered MFGM-insulin into the duodenum; and

4

Fig. 12 is a graph showing how the level of insulin in the blood of diabetic rats varied after they were administered MFGM-insulin into the duodenum.

Fatty globule membranes in mammalian milk are used in the present invention and fatty globule membranes in cow milk are preferred since they can be prepared from easily available materials. The properties, process of production and other aspects of fatty globule membranes in mammalian milk that are to be used in the present invention are described hereinafter.

It is well known that the milk secreted from mammalian animals is a nutritious food. Depending on the species of animals, mammalian milk contains from 2 to as high as 18% of fat, which serves as a source of energy for sucklings. Further, fat that will never mix with water is suspended in milk in the form of fatty globules (0.1 - 17 $\mu$m in dia.) to form a stable suspension. Fatty globules no larger than 1 $\mu$m in diameter occupy at least 80% of the total count in milk but in terms of fat content, such small fatty globules account for only a few percent of the total fat content of milk. The number of fatty globules larger than 6 $\mu$m is also small and they account for only about 2 -3% of the total fat in milk. Most of the fat in milk occurs in fatty globules ranging from 2 to 6 $\mu$m in diameter (3.4 $\mu$m on average) and the fat in these medium-sized globules accounts for 94% of the total fat in milk. Hence, the fatty globules in cow milk, a typical kind of milk, having an extremely large surface area, which can be as large as 2 m$^2$ per gram of fat. Fatty globules having such a vast area can remain stable in suspension because of the presence of milk fatty globule membranes (MFGM), or interfacial layers that separate a highly hydrophobic lipid from ambient water. A common method for separating MFGM from fatty globules consists of obtaining cream by centrifuging milk, washing the cream with water several times, and disrupting fatty globules by mechanical or physical means in a step called "churning". The principle of churning is such that the cream is cooled to harden and embrittle the high-melting triglyceride (HMTG) which is the core of fatty globules. with the brittle fatty globules then being disrupted by vigorous shaking. The fat masses flowing out of the disrupted fatty globules coalesce to form a large lump on the aqueous layer in which MFGM remains dispersed. The thus separated aqueous layer (buttermilk) is dried by some suitable means to obtain MFGM in the form of a white powder. The resulting MFGM is a structural lipoprotein chiefly composed of lipid and protein and is believed to originate from mammary gland cells in view of the process of its synthesis in the body. Stated more specifically, milk fat synthesized in mammary gland cells is covered with mammary gland cell membrane as it is secreted from mammary gland cells and the thus covered fat is secreted into milk. Depending on the specific method employed for separation from cow milk, the yield of MFGM varies from 0.5 to 1.5% per gram of milk fat. At least 90% of MFGM is occupied by protein and lipid, with about 55% of MFGM being recovered as lipid fractions and the remaining 45% as protein fractions.

MFGM differs from other biomembranes in that the lipid fractions contain large amounts of neutral fat called high-melting triglyceride (HMTG). The function of the high-melting triglyceride is not completely clear but since it occurs on the inner surface of MFGM composed of protein and phospholipid in areas where it contacts milk fat, it would play an important role not only in retaining the hydrophobic environment within fatty globules but also in enhancing the strength of the latter. Thus, compared to ordinary bio-membranes having a dual structure, MFGM which contains in it an extremely hydrophobic neutral fat is characterized by a singular structure.

When observed under scanning electron microscope, MFGM is found to have only a two-dimensionally spread membranous structure, not a spherical or hemispherical structure as observed in ribosome.

A. In the present invention, lipid-soluble drugs which are slightly soluble or insoluble in water are suspended by reconstructing MFGM-coated microspheres. In order to coat slightly water-soluble or water-insoluble lipid-soluble drugs with MFGM, the drugs must in principle be in an oily form that will not mix with water. Hence, drugs that are oily at room temperature can be directly coated with MFGM. Solid drugs usually have to be dissolved in appropriate solvents that will not denature proteins, such as vegetable oils a typical example of which is olive oil. However, solid drugs in powder form can be directly mixed with MFGM and suspended in water in the absence of any of the solvents described above. For the purpose of the following discussion, liquid drugs or drug solutions will be called "oils". Emulsions having oils or powders suspended in water with the aid of MFGM may be prepared by the following procedure. First, MFGM is dispersed in a neutral buffer solution; then, an oil is added and emulsified by either mechanical means (e.g. various types of homogenizers) or physical means (e.g. application of ultrasonic waves). This procedure completes the reconstruction of MFGM coated microspheres. When a homogenizer is used to reconstruct MFGM coated microspheres, principal causes of variations are the speed of homogenizer, time, temperature, the concentration of MFGM, the concentration of the oil globules to be coated, and pH, and these parameters can be properly selected. For instance, the concentration of MFGM, or the amount in which MFGM is added to a drug, suffices to be at least 1 wt%. By adding an increased amount of MFGM, not only the emulsion activity and stability of the suspension of MFGM microspheres but also its foam stability can be enhanced.

From a practical view-point, MFGM is preferably added in amounts ranging from 2 to 8 wt%. General conditions for the preparation of MFGM suspensions or emulsions are described below.

General Conditions of MFGM Emulsion Preparation

MFGM (270 mg) is dispersed in a 10 mM sodium phosphate buffer solution (pH, 7.0) to make a total volume of 10 ml. A drug containing oil (2.5 g) is collected in a Pyrex test tube (2.5 × 17 cm) and 7.5 ml of the buffer solution (containing ca. 200 mg of MFGM) is added, followed by heating at 45° C for 5 minutes. The content of MFGM is 80 mg per gram of the oil. With continued heating, the mixture is stirred with a Polytron homogenizer (ST 20 shaft) at a maximum speed (mark 11) for 1 minute to obtain an emulsion. A very stable emulsion (stability factor, 1) can be obtained when the content of MFGM is 80 mg per gram of the oil.

The so obtained emulsion may be converted to a powder form, as required, under the following conditions:

(1) solvent (neutral fat): a neutral fat that is solid or semi-solid at room temperature is selected as a solvent for dissolving the lipid-soluble drug;

(2) extender: milk protein, lactose, etc.

When drug-filled MFGM microspheres are mixed with extender (2) and spray- or freeze-dried, a stable powder containing MFGM coated microspheres can be obtained.

B. The present invention can also be used to formulate various physiologically active peptides in dosage form, and illustrative physiologically active peptides include: insulin, oxytocin, vasopressin, human pituitary gonadotropin, human chrionic gonadotropin, growth hormone, corticotropin, calcitonin, gonadotropic hormone releasing hormone, thyrotropic hormone releasing hormone, interferon-$\alpha$, interferon-$\beta$, erythropoietin, colony stimulating factors GM-CSF, G-CSF and M-CSF, epithelium growth factor (EGF), nerve cell growth factor (NGF), somatomedins, batroxobin, prourokinase, tissue plasminogen activator, staphylokinase, superoxide dismutase, growth hormone releasing factor, somatostatin, atrial sodium diuretic peptides, protein C, protein S, pulmonary surfactants, lipocortin, interleukin 1, interleukin 2, interleukin 3, tumor necrosis factor (TNF), lactoferrin, transferrin and immunoglobulin.

In order to formulate these physiologically active substances in dosage form, they are first dissolved or suspended in water, and after adding a neutral fat and mammalian milk fatty globule membranes (MFGM), the mixture is emulsified by either mechanical or physical means. The neutral fat and MFGM are advantageously added together with a protease inhibitor. While protease inhibitors that can be used are in no way limited, pepstatin and leupeptin may be mentioned as specific examples. Water in which the physiologically active peptides are to be dissolved or dispersed may be replaced by a neutral buffer solution. Phosphate or tris hydrochloride buffer solutions having a pH of 7 may be used as neutral buffer solutions. The neutral fat to be used in suspending physiologically active peptides according to the present invention may be properly selected from among those which are liquid or semisolid at room temperature, and octyldecyltriglyceride (artificial neutral fat), vegetable oils (e.g. coconut oil), butteroil and animal or vegetable oils containing unsaturated fatty acids may be mentioned as specific examples. If insulin is used as a physiologically active peptide, coconut oil may be mentioned as an advantageous neutral fat in view of its high solubility.

Physiologically active peptides can be dispersed either by mechanical means such as various types of homogenizers or by physical means such as the application of ultrasonic waves. Mammalian milk fatty globule membranes (MFGM), say, cow milk fatty globule membranes, are preferably added in amounts ranging from 2 to 10% (w/w) of the neutral fat used.

The emulsion obtained by the method described above may be converted to a powder form, as required, by a process which comprises adding a suitable extender, such as lactose, whey protein, casein or microcrystalline wax, to the emulsion and then freeze-drying or spray-drying the mixture. The thus prepared powder itself is chemically stable and can be formulated in a desired dosage form such as tablet or granule.

Compared to synthetic surfactants, MFGM used in the present invention is characterized by its ability to emulsify neutral fats in an efficient way and by its utmost safety. Synthetic surfactants are a kind of cytotoxins having a hemolytic action and if taken into the body in large quantities, they can cause severe diarrhea resulting from the separation or dislodging of the mucosa of the digestive tract, the occurrence of which phenomenon is by no means rare. On the other hand, MFGM has a surface derived from cell membranes and is very safe to use in the absence of deleterious effects on cell membranes that could be caused by synthetic surfactants. In the present invention, MFGM may be replaced by MFGM-containing dairy products such as buttermilk and cream.

The following examples are provided for the purpose of further illustrating the present invention but are in no way to be taken as limiting.

Example 1 : Measurement of Solubilizing Ability

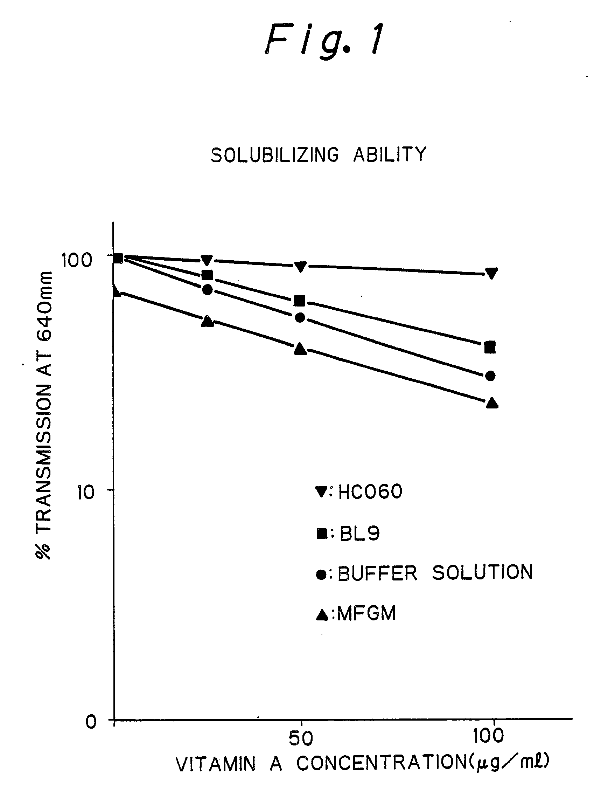

MFGM (by courtesy of Bio-oriented Technology Research Advanced Institute, Chugai Seiyaku Kabushiki Kaisha), HCO 60 (Nikko Chemicals Co., Ltd.) and BL 9 (Nikko Chemicals Co., Ltd.) were suspended or dissolved in water at a concentration of 100 μg/ml. To the resulting suspensions or solutions, vitamin A was added at three different concentrations (25, 50 and 100 μg/ml) and sonicated with an ultrasonic cell disrupter (Ohtake Seizaisho K.K.) at 30 W for 5 minutes. The light transmittance of each sample was measured and the results were as shown in Fig. 1, from which one can see that whichever solubilizer was used, transmittance decreased (i.e. turbidity increased) with the increasing concentration of vitamin A. Upon closer examination, the increase in turbidity was low when HCO 60 was used, whereas the behavior with BL 9 and MFGM was substantially the same as when a buffer solution alone was used. It was therefore clear that MFGM was little effective solubilizing vitamin A. In Example 1, triolein was used as an oil.

Example 2 : Measurement of Emulsifying Ability

Preparation of emulsions:

One gram of an oil containing 12 mg of vitamin A was combined with 19 g of a sodium phosphate buffer solution containing 80 mg/ml of MFGM. After heating at 40 - 45°C for 5 minutes, the mixture was stirred with a Polytron homogenizer at a maximum speed for 1 minute to obtain an emulsion (which contained 5 wt% of the oil).

Additional emulsions were prepared by repeating the same procedure except that 1 g of a oil containing 12 mg of vitamin A was mixed with 19 g of a sodium phosphate buffer solution containing one of the surfactants described below (80 mg/ml) in place of MFGM: lecithin (Sigma Chemical Company), sucrose aliphatic acid ester P-1670 (Mitsubishi Kasei Shokuhin K.K.), Tween 80 (Nikko Chemicals Co., Ltd.) and HCO 60 (Nikko Chemicals Co., Ltd.)

(1) Measurement of emulsion stability

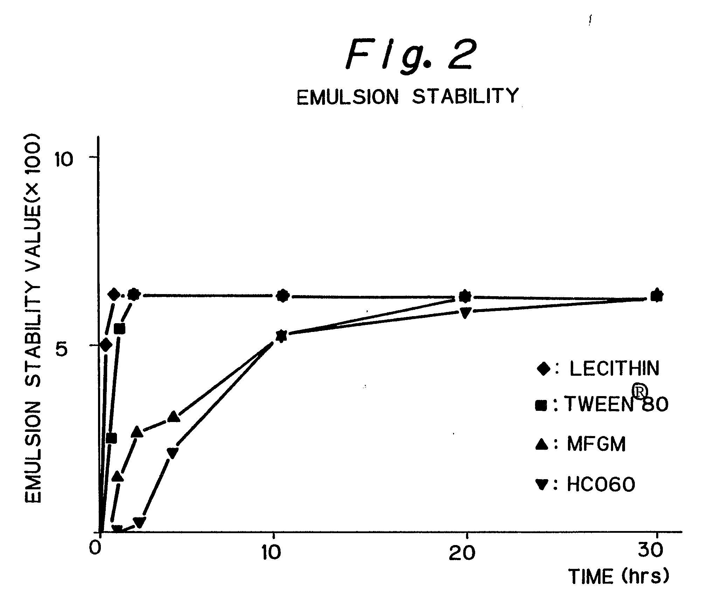

Each of the emulsions was put into a test tube, which was readjusted to a vertical position and left to stand at room temperature. At given time intervals, the length of the cream layer and the overall length of the emulsion layer were measured. On the basis of the measured values, the length of the cream layer divided by the overall length of the emulsion layer was determined to provide a measure of emulsion stability. The results are shown in Fig. 2. In this experiment, triolein was used as an oil.

As one can see from Fig. 2, the emulsion stability values of MFGM were lower than those of lecithin and Tween 80 but comparable to those of HCO 60. This indicates the high emulsion stability of MFGM.

(2) Measurement of emulsion activity

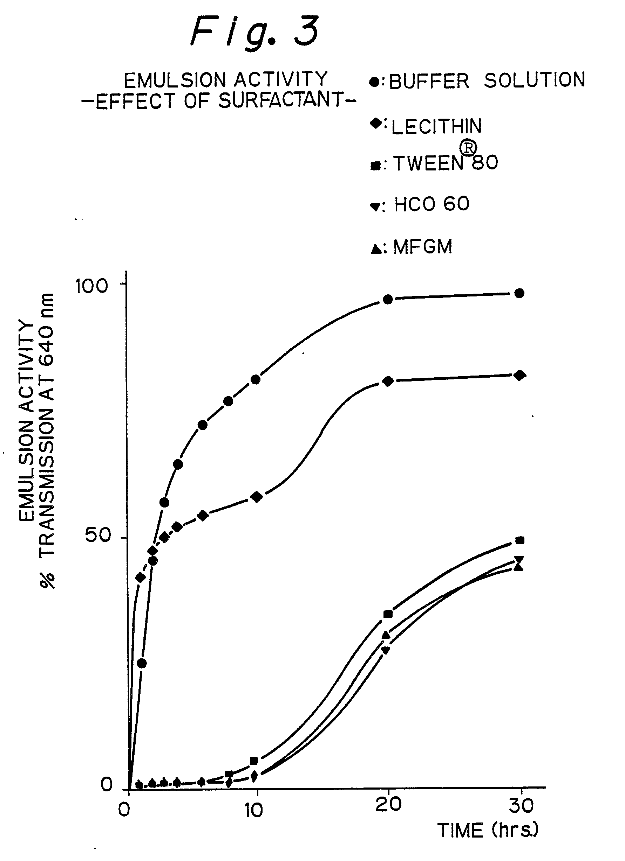

Each of the emulsions was put into a test tube, which was readjusted to a vertical position and left to stand at room temperature. At given time intervals, 0.2-ml portions of each emulsion were collected from the bottom of the test tube and mixed with 3.8 ml of an aqueous solution of 0.1% sodium dodecylsulfate. The light transmittance of each sample was measured under various conditions and the results of measurements are shown in Figs. 3 - 6.

Fig. 3 shows the emulsion activity of various surfactants in emulsions. According to this graph, the emulsion activity of MFGM was lower than those of a buffer solution (indicated by Buffer Solution in the drawing) and lecithin and exhibited substantially the same value as those of Tween 80 and HCO 60 until after 30 hours. The emulsion activity of MFGM remained particularly low until after 10 hours, indicating that MFGM was effective in retaining the stability of emulsions.

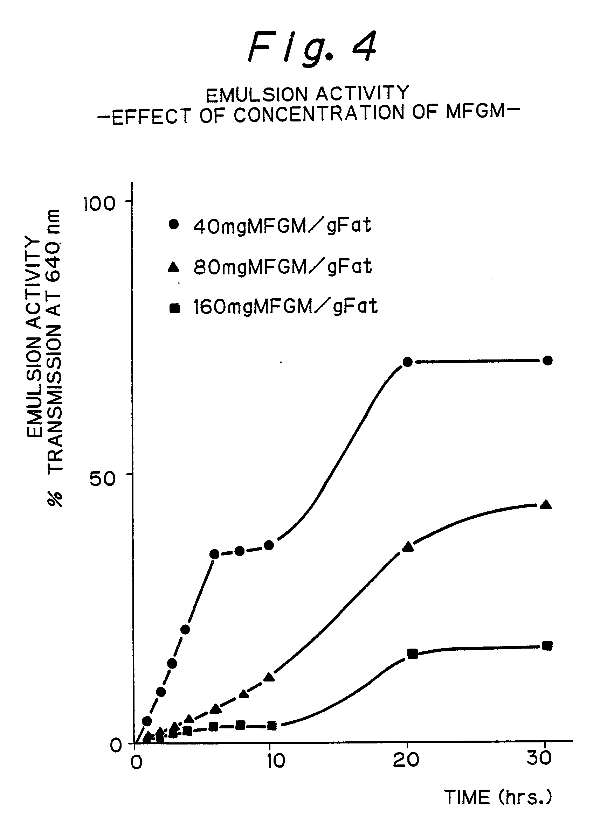

Fig. 4 shows the dependency of emulsion activity of MFGM on concentration. According to this graph, the increase in emulsion activity was rapider when MFGM was added in an amount of 40 mg per gram of oil than when it was added in an amount of 80 mg per gram of oil as in the usual case. On the other hand, the increase in emulsion activity remained low until after 30 minutes when MFGM was added in an increased amount of 160 mg per gram of oil. These data suggest that the higher the MFGM content, the higher the stability of the emulsion.

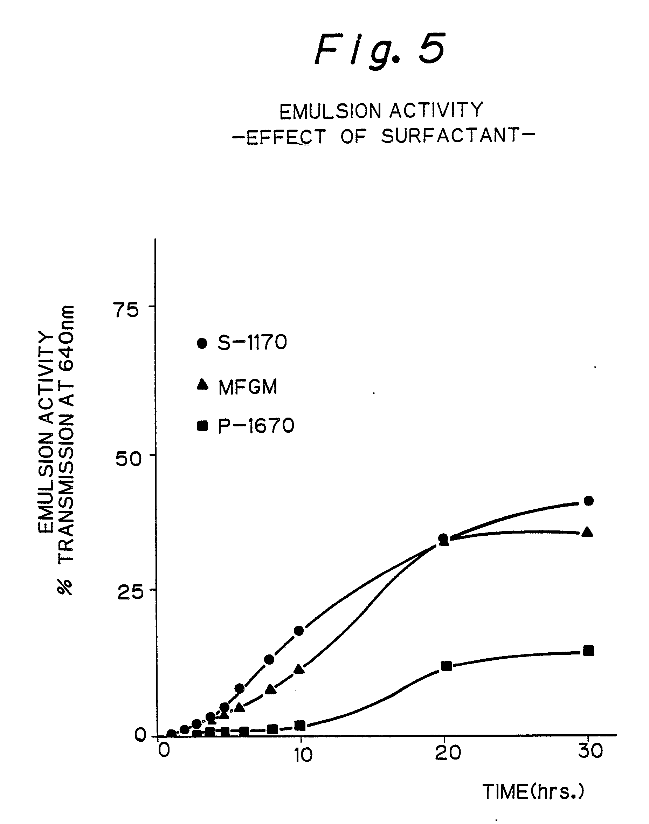

Fig. 5 compares MFGM with sucrose aliphatic acid esters which are considered to be highly safe surfactants in order to evaluate the effect of MFGM on emulsion activity. According to this graph, MFGM caused a greater increase in emulsion activity than P-1670 (a sucrose aliphatic acid ester with HLB of 16 that had a palmitic acid residue bound thereto) but the behavior was substantially the same as that of S-1170 (a sucrose aliphatic acid ester with HLB of 11 that had a stearic acid residue bound thereto).

Triolein was used as an oil in the experiments the results of which are shown in Figs. 3 - 5.

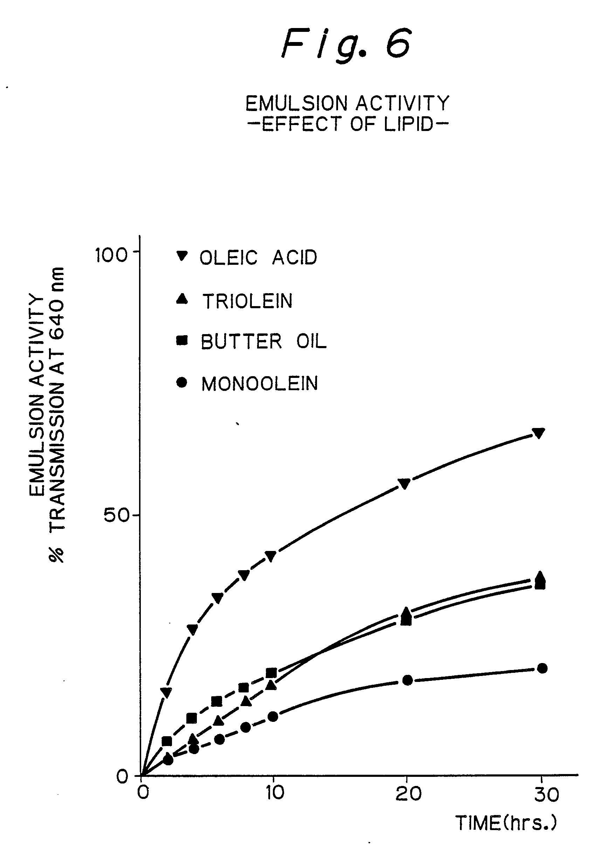

Fig. 6 shows the dependency of emulsion activity on the type of fats or oils including triolein. Besides triolein, oleic acid, monoolein and butteroil were used in the experiment. As it turned out, the emulsion containing monoolein experienced the least increase in emulsion activity whereas the highest increase in emulsion activity occurred when oleic acid was used. Butteroil and triolein showed intermediate and substantially the same values.

Drug-containing MFGM microspheres that were prepared by the method of the present invention were administered into animals and the profiles of drug absorption by the animal body were compared with those of dosage forms prepared by the conventional method.

Example 3 : Effect of MFGM on the Absorption of DDT through Lymphatic Vessels

The test drug was DDT, which is commonly used as a model compound for lipid-soluble drugs when the absorption of orally administered drugs is studied.

First, Wistar male rats were starved overnight and subjected to a surgical operation for inserting cannulas into the duodenum and lymph in the thoracic duct. Using a solution of DDT in olive oil, an emulsion of MFGM containing microspheres was prepared under the conditions already described herein. An MFGM-free DDT solution in olive oil was used as a control. Each of the emulsion and the control was administered into the duodenum of the rats.

The DDT dose was 10 mg/kg in all instances.

After the administration of DDT, lymph flowing out of the thoracic duct was collected for DDT analysis. The results are shown in Table 1.

Table 1

| Effect of MFGM on the Absorption of DDT through Lymphatic Vessels after Administration into the Duodenum | | | | |
|---|---|---|---|---|
| | Olive oil solution | | MFGM-containing microspheres | |
| | 0 - 12 h | 12 - 24 h | 0 - 12 h | 12 - 24 h |
| Absorption ratio (%) | 15.31 + 7.79 | 0 | 41.16 + 14.7** | 0 |
| Number of rats | 8 | 6 | 4 | 4 |

** $p < 0.01$

As is clear from Table 1, when DDT dissolved in olive oil was administered into the duodenum of rats, DDT was absorbed in a total amount of only 15.31% within a period of 12 hours. On the other hand, when the solution of DDT in olive oil that had been reduced to microspheres with the aid of MFGM was administered in a similar way, DDT absorption increased to 41.16%, clearly supporting the great ability of MFGM to promote the absorption of DDT.

Example 4 : Effect of MFGM on the Absorption of Vitamin A through Lymphatic Vessels

Wistar male rats each weighing about 400 g were starved for 24 hours. The starved rats were anesthetized by intraperitoneal administration of pentobarbital. Thereafter, the rats were incised in the belly and silicon cannulas were inserted into the area between the pylorus of the stomach and the duodenum and into the ileocecal area. Through the upper cannula, liquid drugs (5 ml) composed of the emulsions prepared in Example 1 were injected into the loop of small intestines (duodenum, jejunum and ileum) and the cannula was closed with a clamp.

Blood and lymph fluid were collected from the hip artery and the lymph in the thoracic duct at given time intervals and the concentrations of vitamin A in the two fluids were measured. A similar experiment was conducted on biliary fistula rats from which bile was excreted through a cannula inserted into the bile duct, and comparison was made with intact rats which had not received such a treatment.

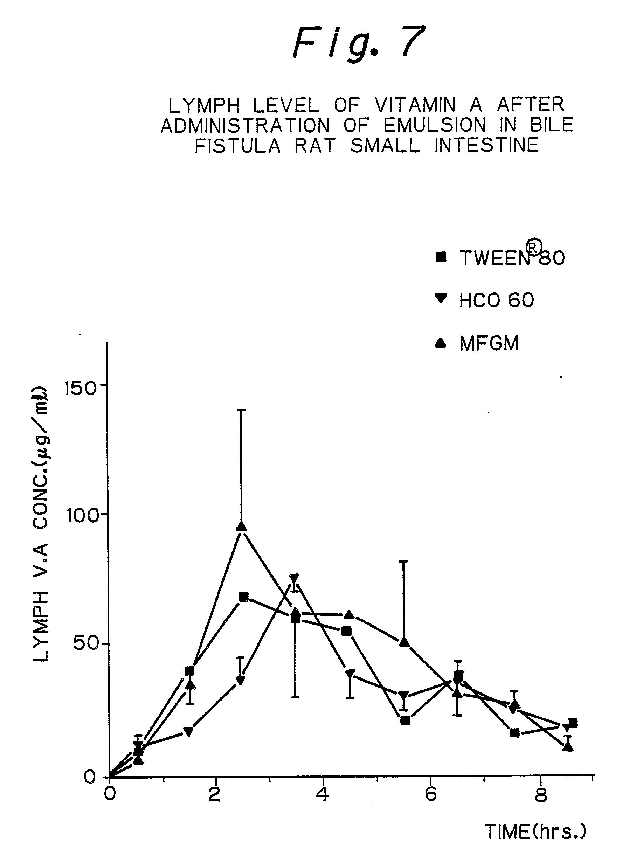

The dose of vitamin A was 15 mg per rat. After extraction with cyclohexane, the concentration of vitamin A in each sample was measured by fluorometry at 480 nm with fluorescence excited at 340 nm. The results are shown in Figs. 7 - 9. After being absorbed by the small intestines, vitamin A forms chylomicron, the greater part of which is known to be transferred into the lymph system, not the blood system. In the experiments conducted in Example 4, vitamin A was also hardly detectable in the blood, so the effectiveness of MFGM was evaluated in terms of the concentration of vitamin A in lymph fluid.

Fig. 7 shows the concentration profile of vitamin A as obtained when emulsions prepared by using sesame oil were administered into the loop of small intestines in the biliary fistula rats. According to the graph, at no time intervals were observed significant differences in concentration between the case where MFGM was used and the case where synthetic surfactants, Tween 80 and HCO 60, were used. In all instances, the concentration of vitamin A in lymph reached a maximum level 2.5 - 3.5 hours after the administration but the highest level was attained when MFGM was used.

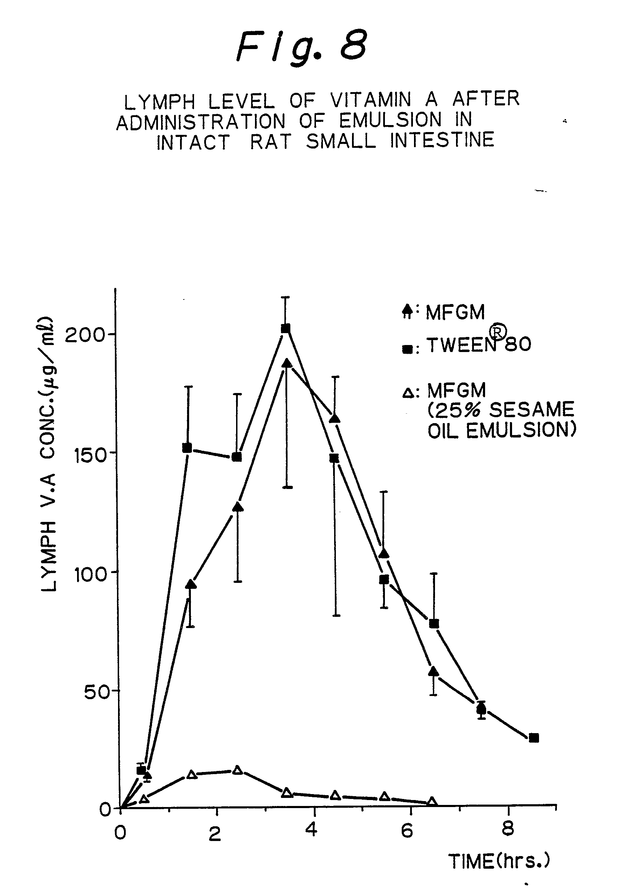

Fig. 8 shows the concentration profile of vitamin A as obtained when emulsions were administered into the loop of small intestines in the intact rats under the same conditions as those employed to construct the graph in Fig. 7. According to Fig. 8, the peak value was two to three times as high as the initial value irrespective of whether MFGM or Tween 80 was used as a solubilizing agent, and this indicates the increased transfer of vitamin A into lymph. Fig. 8 also shows that when the oil content was increased from 5 to 25%, the concentration of vitamin A in lymph dropped to very low levels and this clearly shows that the absorption of vitamin is largely dependent on the oil content of an emulsion.

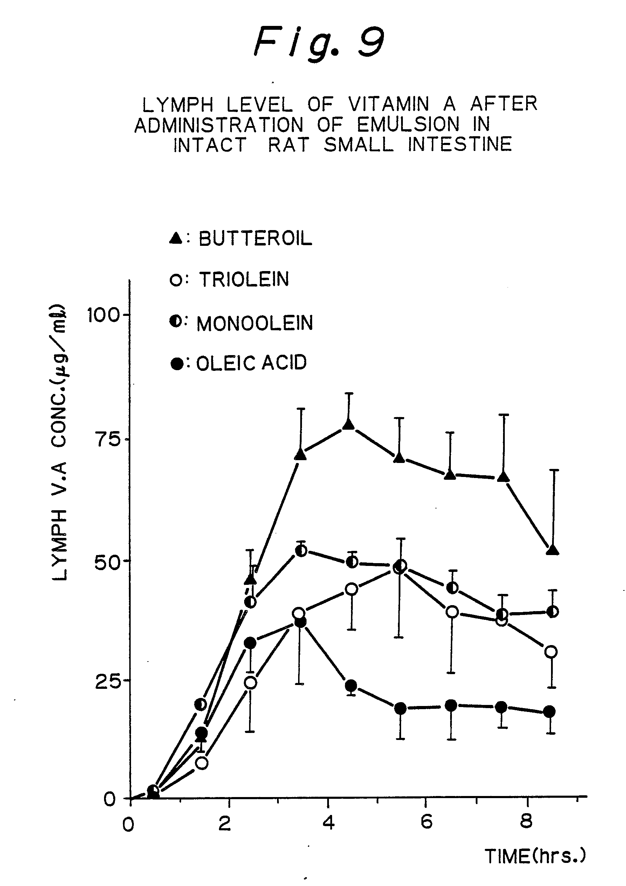

Fig. 9 shows how the concentration of vitamin A in lymph varied depending upon the type of fat or oil used in emulsions. The dose of vitamin A was 2.5 mg per rat. All the emulsions tested had a fat or oil content of 5%. According to Fig. 9, the concentration of vitamin A in lymph was the highest when butteroil was used, whereas the lowest level was observed when oleic acid was used. It was therefore clear that the use of an emulsion in butteroil that was very close to the state of naturally occurring milk fatty globule membranes was effective for the purpose of enhancing the absorption of vitamin A.

While the foregoing data shows the effects of MFGM on the absorption of DDT and vitamin A through lymphatic vessels, it should be mentioned that MFGM was also found to be effective in promoting the absorption of other lipid-soluble drugs. For example, even such a compound as vitamin E that was oily at room temperature could be absorbed through digestive tracts in a significantly increased amount when it was reduced to microspheres with the aid of MFGM. This was demonstrated by the following experiment: when a solution of vitamin E in cottonseed oil and microspheres of that solution which were prepared with the aid of MFGM were administered orally to rats, the latter was absorbed in an amount 2.6 times as great as the absorption of the former. This ability of MFGM to promote the absorption of lipid-soluble drugs when they were reduced to microspheres under the same conditions was also observed with lipid-soluble vitamins such as vitamin D and vitamin K, and with isoprene compounds such as ubiquinone. Similar effects were also observed with various other lipid-soluble drugs such as probucol, avan, isoflavone and prostaglandins.

Drugs that are slightly soluble in both water and organic solvents but the partition coefficients of which are much more favorable to an organic solvent (e.g. octanol) than water can also be absorbed in a significantly enhanced way if they are administered after being reduced to microspheres with the aid of MFGM. Hydrocortisone is an example of such drugs and the following experiment was conducted on hydrocortisone.

Example 5 : Effect of MFGM on the Absorption of Hydrocortisone in to the Body

Wistar male rats each weighing ca. 300 g were starved overnight. Thereafter, their skin above the right common carotid artery was incised and a cannula was inserted into the artery. In a separate step, a solution of tritium ($^3$H) labelled hydrocortisone in cottonseed oil was reduced to microspheres with the aid of MFGM according to the general conditions of emulsion preparation already described herein. The so prepared emulsion was administered orally to the rats. As a control, hydrocortisone reduced to a transparent micellar form with the aid of surfactant Tween 80 was used. The dose of hydrocortisone was 1.5 mg/kg. After hydrocortisone administration, blood samples were taken at regular time intervals. Feces and urine were collected and the rats were killed 49 hours after the administration. The digestive tracts, their contents and various organs were separated and the content of hydrocortisone in each site was determined by radioactivity measurement.

When lipid-soluble drugs are treated with Tween 80, an apparently transparent solution will form to give the impression that the drugs have been dissolved. In fact, however, the drugs are not dissolved at the molecular level and the greater part of them remains as fine micelles the diameter of which is smaller than the wavelength of light, thus allowing transmission of light. It is well known that when such lipid-soluble drugs in a transparent micellar form are administered, the rate and quantity of drug absorption increase significantly for the following two probable reasons:

(1) the diameter of micelles is smaller than the wavelength of light, so the surface area of drug particles increases markedly;

(2) the surfactant used to form micelles causes temporary damage to the mucosa of the small intestines, so the latter will not provide a barrier against increased drug permeation.

Hence, the effectiveness of MFGM to promote hydrocortisone absorption was evaluated in Example 5, against the test conditions (control) that allowed hydrocortisone to be absorbed at the highest rate.

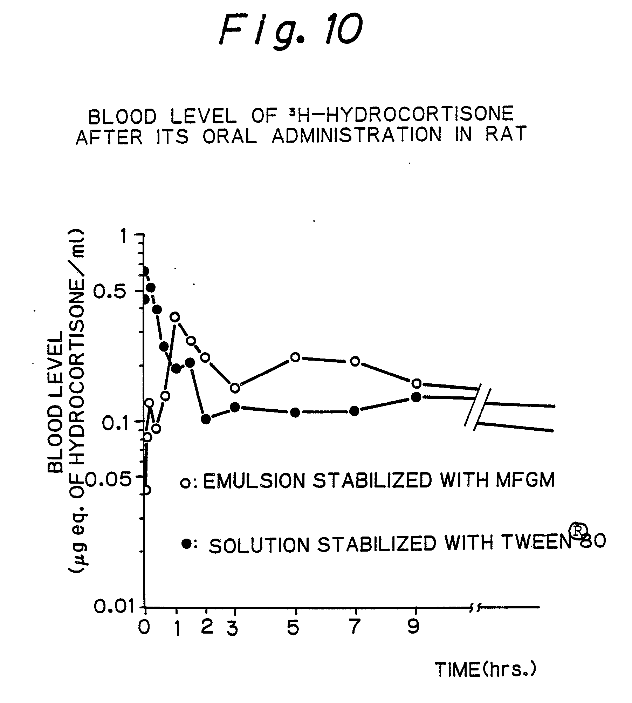

The results of the test conducted in Example 5 are shown in Fig. 10 and Table 2. Fig. 10 shows the concentration profile of tritium labelled hydrocortisone in blood after its administration to the rats. When Tween 80 was used as a solubilizer, a maximum blood level was reached 5 minutes after the administration and the maximum blood level attained was a little less than twice as high as the level attained when hydrocortisone was administered in the form of MFGM-containing microspheres. In the latter case, it took 60 minutes for a maximum blood level to be reached and the peak height was low. However, in terms of AUC, the MFGM treated group was either comparable to or superior to the control group. Consequently, it can be concluded that when hydrocortisone was administered in the form of MFGM-containing microspheres, the AUC attained was at least comparable to that of hydrocortisone micelles prepared by solubilization with Tween 80 and which are held to exhibit the highest blood level and AUC of all the pharmaceutical preparations available today for oral administration.

Table 2 shows the effects of MFGM on the residence of hydrocortisone in the body. It is suggested from this table that when hydrocortisone is administered in the form of MFGM-containing microspheres, the drug is absorbed by the body through a different route than when it is administered in the form of micelles prepared with the aid of Tween®80.

Table 2

| Effects of MFGM on the Residence of Hydrocortisone in the Body | | |
|---|---|---|
| | MFGM (n = 3) | Tween®80 (n = 2) |
| | Excretion (% of dose) | |
| urine | 9.6 ± 0.2 | 11.6 ( 8.5, 14.8) |
| feces | 35.3 ± 13.5 | 39.0 (32.3, 45.6) |
| subtotal | 44.9 ± 13.7 | 50.6 (40.8, 60.4) |
| | Distribution (% of dose) | |
| stomach | 0.23 + 0.25 | 0.15 ( 0.21, 0.08) |
| small intestines | 0.75 ± 0.47 | 3.98 ( 5.54, 2.41) |
| large intestines | 0.06 ± 0.04 | 0.38 ( 0.72, 0.03) |
| appendices | 9.25 ± 3.07 | 18.96 (17.70, 20.21) |
| contents of the stomach | 0.52 ± 0.58 | 0.34 ( 0.60, 0.07) |
| contents of the small intestines | 3.27 ± 1.37 | 12.71 (16.68, 8.54) |
| contents of the large intestines | 1.62 ±.1.64 | 2.59 ( 5.14, 0.04) |
| subtotal | 15.69 ± 2.34 | 39.09 (46.79, 31.38) |
| grand total | 60.56 ± 11.72 | 89.69 (87.59, 91.78) |
| residual in the body | 39.44 ± 11.72 | 10.31 (12.41, 8.22) |

As is clear from Table 2, the residual amount of hydrocortisone in the rats as calculated by subtracting the sum of excretion and the residual in the digestive tracts from the total amount of administration was 12.41% in the Tween 80 group but 53.12% in the MFGM group, which was 4.3 times as high as the accumulation of the drug in the Tween® 80 group. This great difference in drug accumulation in the body could be explained by the difference in the route of drug absorption. When administered in the form of micelles prepared with the aid of Tween 80® hydrocortisone absorbed by the mucosa in the intestinal tract will probably pass through the portal vein and the liver to flow into the systemic circulation. On the other hand, when administered in the form of MFGM-containing microspheres, hydrocortisone adhering to the mucosa in the intestinal tract would be taken into lymphatic vessels and flow into the vein through thoracic lymphatic vessels.

To summarize the foregoing discussion, MFGM offers the following advantages when it is used to formulate, in dosage form, drugs such as hydrocortisone that are slightly soluble in both water and organic solvents but the partition coefficients of which are much more favorable to organic solvents than water:

(1) hydrocortisone microspheres prepared with the aid of MFGM are comparable in absorption ratio to micelles that are prepared by solubilization with synthetic surfactants and that exhibit very high absorption rate and ratio;

(2) unlike synthetic surfactants, MFGM will not damage the mucosa in the intestinal tract;

(3) because of a different route of absorption, an MFGM-treated drug will stay within the body for a significantly prolonged period to work as a long-lasting drug.

Example 6

Coconut oil (3 ml) was added to 3.5 ml of a 10 mM phosphate buffer solution (pH 7.0) containing 80 mg of MFGM. Further, 6 ml of a human insulin solution (40 U/ml) was added and the mixture was heated at 45°C for 5 minutes. The heated mixture was stirred for 1 minute with a Polytron homogenizer to emulsify the coconut oil completely. The resulting emulsion contained 19.2 U of insulin per milliliter. The preparation could be immediately administered perorally but in order to improve its keeping quality, 7.5 g of lactose and 1.2 g of whey protein were added and the mixture was freeze-dried to form a powder. The powder contained insulin in an amount of 20 U/g.

Example 7

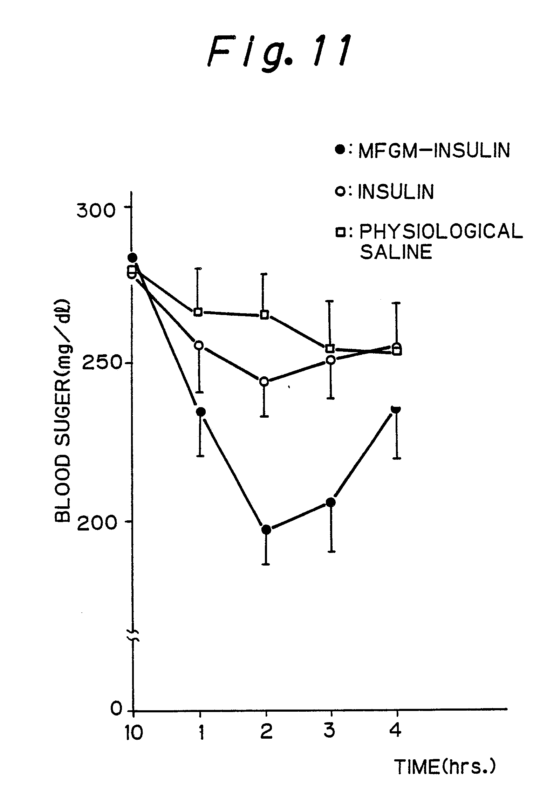

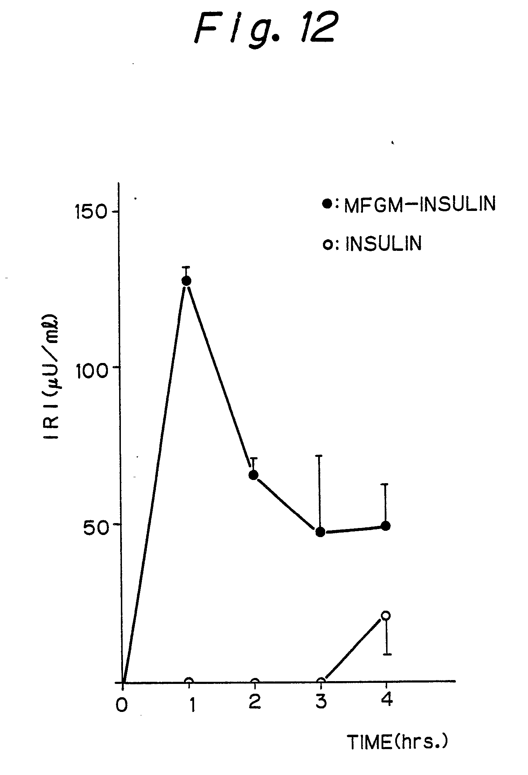

A solution (pH 4.0) of streptozotocin in 10 mM citric acid was injected in an amount of 60 mg/kg into 7-week old male Donryu rats each weighing 250 g through the tail vein to construct insulin-dependent diabetic models. The rats were starved for 12 hours one week after the intravenous injection of streptozotocin and divided randomly into three groups, the MFGM/insulin treated group, the insulin-treated group and the control group administered physiological saline solution. The experiment started with incising the rats in the belly under anesthetization with nembutal. A catheter was inserted into the duodenum and 1 ml of insulin (40 U) or physiological saline solution was injected through the catheter into the intestinal tract. At given intervals (1, 2, 3 and 4 hours) after the injection, blood samples were taken through the tail vein and the levels of blood sugar and immunoreactive insulin (IRI) were measured. As shown in Fig. 11, the initial blood sugar level was about 280 mg/dl in each group. The 1- and 2-hour values for the insulin-treated groups were lower than those for the control group but the difference was not significant. The blood sugar level of the MFGM/insulin treated group decreased rapidly until after 2 hours passed and the 2- and 3-hour values were significantly lower than the corresponding values for the control group. The data shown in Fig. 11 represents the blood sugar level of 9 or 10 diabetic rats in terms of mean value ± standard deviation. The level of blood IRI in the control group as measured by radio-immunoassay (Dinabbot Laboratories) was below the detection limit at each point of time. The blood IRI in the MFGM/insulin treated group was not detectable before injection but after 1 hour, the blood IRI increased up to 130 µU/ml and decreased gradually at 2 hours and thereafter. The 1- and 2-hour values of blood IRI for this group were significantly (p < 0.01) higher than the corresponding values for the insulin-treated group, and the 3-hour value was also significantly (p < 0.05) higher than the corresponding value for the insulin-treated group. The difference between the two groups was of a factor of at least 100 in terms of AUC. The data shown in Fig. 12 represents the blood insulin level of 9 or 10 diabetic rats in terms of mean value ± standard deviation. The blood insulin level in the control group was below the detection limit at each point of time and is omitted from Fig. 12. If the amount of blood is assumed to be a thirteenth of the body weight, the insulin absorption ratio of the MFGM/insulin treated group at 1 hour after injection is about 5%, and considering the fact that one third of the absorbed insulin is captured in the liver, the data suggests that about 8% of the insulin administered was absorbed through the intestinal tract. When administered by the prior art method, insulin is absorbed through the intestinal tract at ratios of up to 1% in the best case, and which are typically no more than 0.1%. Hence, the MFGM/insulin treated group was capable of absorbing orally administered insulin through the intestinal tract at ratios which were 50 - 100 times as high as in the case where it was administered by the prior art method.

Example 8

Male Sprague-Dawley rats each weighing 300 g were injected intravenously with 50 mg/kg of streptozotocin as in Example 7 to develop diabetes. One week after the injection of streptozotocin, the rats were divided randomly into three groups, one being the control group and the other two being the insulin-treated group and insulin/MFGM treated groups, respectively. After being starved overnight, the control group (n = 6) was administered physiological saline solution perorally; another group (n = 6) was administered orally 40 U of insulin in the form of an aqueous suspension of the MFGM/insulin powder prepared in Example 1; the third group (n = 6) was administered 40 U of insulin perorally. The dose of the solution administered was 1 ml per 100 g of body weight. The rats were housed in Bollman cages and at given time intervals, blood samples were taken through the tail vein for the measurement of blood sugar and IRI levels.

Table 3

| Blood Sugar and IRI levels in Streptozotocin Diabetic Rats | | | | | | |
|---|---|---|---|---|---|---|
| Group | | Time (min.) | | | | |
| | | 0 | 30 | 60 | 90 | 120 |
| Control | blood sugar (mg/dl) | 289±20 | 296±23 | 271±19 | 272±27 | 288±32 |
| | IRI (μU/ml) | nd | nd | nd | nd | nd |
| Insulin-treated | blood sugar (mg/dl) | 290±16 | 293±23 | 281±22 | 276±20 | 272±18 |
| | IRI (μU/ml) | nd | nd | nd | nd | 8±3* |
| Insulin/MFGM-treated | blood sugar (mg/dl) | 285±12 | 155±16* | 133±13* | 168±17* | 162±21* |
| | IRI (μU/ml) | nd | 99±11* | 153±8* | 137±10* | 106±9* |
| Data shown in terms of mean ±S.D. nd: not detected. | | | | | | |

* $p < 0.01$ in Student's paired t-test

As is clear from Table 3, it was only in the MFGM/insulin treated group that high levels of insulin were detected from blood while the blood sugar level decreased. In the insulin-treated group which was administered only the same amount of insulin solution, insulin was little detected from blood and no significant drop in the blood sugar level was oserved. It is therefore clear that insulin which is emulsified with the aid of cow milk fatty globule membranes and an oil or fat can be absorbed in large quantities through the intestinal duct even if it is administered in a powder form prepared after adding cow milk ingredients to the emulsion.

Example 9

Twenty-four healthy Wistar male rats each weighing ca. 200 g were starved overnight and divided randomly into three groups: the first group was a control group (n = 8), the second group was an insulin-treated group (n = 8) and the third group was an oil-emulsified insulin-treated group (n = 8). The oil-emulsified insulin was prepared by the following method: 10 ml of cream separated from fresh cow milk was added to 10 ml of a human insulin solution (40 U/ml) and the ingredients were completely mixed. The rats of the first, second and third groups were orally administered physiological saline solution, insulin solution and oil-emulsified insulin, respectively. The dose of the solution administered was 1 ml per 100 g of body weight, and the dose of insulin was 20 U/kg. The rats were housed in restraining cages and blood samples were taken through the tail vein immediately before administration, as well as 30, 60, 120 and 180 minutes after the administration. The levels of blood sugar and IRI in the samples were measured. The results are shown in Table 4, from which one can see that only when insulin was administered in admixture with cream did insulin appear in large amounts in blood, with a consequent drop in the blood sugar level.

Table 4

| Blood Sugar and IRI Levels in Healthy Rats Administered Oil-emulsified Insulin | | | | | | |
|---|---|---|---|---|---|---|
| Group | | Time (min.) | | | | |
| | | 0 | 30 | 60 | 120 | 180 |
| Control | blood sugar (mg/dl) | 75 ± 5 | 76±4 | 75±6 | 73±5 | 69±4 |
| | IRI (μU/ml) | 7 ± 5 | 7±3 | 8±5 | 8±3 | 7±5 |
| Insulin | blood sugar (mg/dl) | 76 ± 7 | 69±8 | 65±8 | 67±9 | 69±11 |
| | IRI (μU/ml) | 7 ± 4 | 8±3 | 7±3 | 8±4 | 11±7 |
| Oil-emulsified insulin | blood sugar (mg/dl) | 75 ± 4 | 60±3* | 53±4* | 55±4* | 64±3* |
| | IRI (μU/ml) | 7 ± 1 | 43±12* | 77±10* | 64±8* | 27±5* |
| Data shown in terms of mean ±S.D. | | | | | | |

* p < 0.01 in Student's paired t-test

## Example 10

An experiment was conducted by administering orally a human growth hormone (prepared by genetic engineering) into Wistar male rats. Wistar male rats each weighing about 200 g were cannulated at the tail vein under anesthetization with ether and restrained in Bollman cages. A human growth hormone (1 mg) was dissolved in 14 ml of phosphate buffer saline (PBS, pH 7.4) and heated at 40°C after addition of MFGM (240 mg). To the heated solution, 6 ml of coconut oil that had been heated at 40°C was added and the mixture was homogenized with a Polytron® homogenizer at maximum speed for 1 minute to prepare an emulsion of the growth hormone (50 μg/ml). PBS and a PBS solution of growth hormone (50 μg/ml) were used as controls. The rats were divided into three groups each consisting of 6 animals. The PBS control group was administered PBS perorally. The growth hormone control group was administered a PBS solution of growth hormone perorally. The growth hormone emulsion group was administered the emulsion of growth hormone perorally. The dose of administration was 2 ml per rat in each group. Blood samples (0.3 ml) were taken through the tail vein at regular time intervals and the level of human growth hormone in each sample was determined by two-antibody radioimmunoassay.

Table 5

| Concentration of Growth Hormone in the Blood of Rats Administered Emulsions of Human Growth Hormone (means ± S.D.) | | | | | |
|---|---|---|---|---|---|
| | Growth hormone in blood (ng/ml) | | | | |
| | 0 | 30 min. | 60 min. | 90 min. | 120 min. |
| Control | nd | nd | nd | nd | nd |
| Growth hormone treated | nd | nd | nd | 4.8±2.2 | 7.3±4.0 |
| Emulsion treated | nd | 105.2±35.8* | 857.2±187.2* | 867.7±245.6* | 1085.9±300.7* |
| nd: below detection limit | | | | | |

* p < 0.001 in Student's t-test

When administered in the form of an emulsion, human growth hormone appeared in blood at high concentration at 30 minutes after the administration and a maximum value was reached 2 hours after the administration. Human growth hormone was not detected from the blood of the PBS control group. When

14

human growth hormone was administered perorally as a solution in PBS, the hormone appeared in a very small amount in blood at 90 minutes after the administration but the ratio of its absorption through the intestines is estimated to be no more than a hundredth of the value for the emulsion. The superiority of the emulsion is therefore obvious.

Example 11

Wistar male rats each weighing ca. 200 g were cannulated at the duodenum and tail vein under anesthetization with either and restrained in Bollman cages. Eel calcitonin (1 mg) was dissolved in 7 ml of phosphate buffer saline (pH 7.4) and the solution was heated at 40° C after addition of MFGM (120 mg). To the heated solution, 3 ml of butteroil preliminarily heated at 40° C was added and the mixture was sonicated for 2 minutes to prepare a calcitonin emulsion (100 μg/ml). The calcitonin emulsion (0.5 ml) was injected into the duodenum through the cannula and blood samples (0.3 ml) were taken through the tail vein at regular time intervals. The level of eel calcitonin in each blood sample was determined by two-antibody radioimmunoassay. A control group was injected with 0.5 ml of PBS(-), and a calcitonin control group was injected with 0.5 ml of a solution of calcitonin in phosphate buffer saline (100 μg/ml). Blood samples were taken from these control-groups according to the same schedule as in the case of the emulsion treated group. Thirty minutes before the administration of calciton, calcium chloride in physiological saline was administered intraperitoneally in an amount of 60 mg/kg in order to stimulate the secretion of endogenous calcitonin. Each rat group consisted of 6 animals.

Table 6

| Concentration of Calcitonin in the Blood of Rats Administered Emulsions of Calcitonin (means ± S.D.) | | | | | |
|---|---|---|---|---|---|
| | Calcitonin in blood (ng/ml) | | | | |
| | 0 | 30 min. | 60 min. | 90 min. | 120 min. |
| Control | 0.1±0.05 | 0.3±0.24 | 0.5±0.18 | 0.7±0.56 | 0.5±0.33 |
| Calcitonin treated | 0.1±0.08 | 0.7±0.09 | 0.7±0.22 | 0.8±0.31 | 0.6±0.40 |
| Emulsion treated | 0.1±0.03 | 15.3±5.68 | 58.9±8.85 | 75.2±4.02 | 67.7±5.63 |

As is clear from Table 6, the concentration of calcitonin in the blood of rats administered calcitonin in emulsion form was markedly higher than the concentrations in the two control groups, verifying the fact that calcitonin in the emulsion was absorbed in a significant amount through the intestinal tract.

Example 12

Wistar male rats each weighing ca. 200 g were cannulated at the portal vein under anesthetization with ether and restrained in Bollman cages. Lactoferrin (0.1 g) separated from cow milk was dissolved in 14 ml of phosphate buffer saline (pH 7.4) and the solution was heated at 40° C after adding 240 mg of MFGM. To the heated solution, 6 ml of butteroil preliminarily heated at 40° C was added and the mixture was sonicated for 2 minutes to prepare an emulsion of lactoferrin (5 mg/ml). As a control, a PBS solution of lactoferrin (5 mg/ml) was prepared. The lactoferrin solution or emulsion was injected in 2-ml portions into the stomach through a gastric tube. Blood samples (0.3 ml) were taken through the portal vein at regular time intervals and the level of bovine lactoferrin in each blood sample was determined by two-antibody radioimmunoassay. Each rat group consisted of 6 animals.

Table 7

| Concentration of Bovine Lactoferrin in the Portal Venous Blood of Rats Administered Emulsions of Lactoferrin (means ± S.D.) | | | | | |
|---|---|---|---|---|---|
| | Lactoferrin (μg/ml) | | | | |
| | 0 | 30 min. | 60 min. | 120 min. | 180 min. |
| Control | nd | nd | nd | nd | nd |
| Lactoferrin treated | nd | 1.0±0.08 | 0.7±0.09 | 0.7±0.22 | 0.8±0.31 |
| Emulsion treated | nd | 27.5±5.33* | 74.1±6.44* | 86.2±9.00* | 85.9±10.25* |
| nd: below detection limit | | | | | |

* p < 0.001

As is clear from Table 7, the appearance of lactoferrin in blood was negligible when it was administered as a solution in PBS whereas it appeared in high concentrations in blood when it was administered orally as an emulsion. It is therefore certain that bovine lactoferrin having a molecular weight of $8.7 \times 10^4$ is absorbed by the body through digestive tracts when it is administered orally as an emulsion together with cow milk fatty globule membranes.

Example 13

Eight grams of bovine immuloglobulin having a neutralizing antibody titre of 1:31250 against rotavirus (strain NCDV; type 6 serum) was dissolved in 400 ml of phosphate buffer saline (PBS, pH 7.4) and the solution was heated at 40° C while 16 g of milk fat and 800 mg of cow milk fatty globule membranes was added. The mixture was then homogenized with a Polytron homogenizer for 2 minutes. The resulting emulsion (200 ml) was administered orally to four healthy adult male volunteers. Blood samples (5 ml) were taken immediately after the administration, as well as 30 minutes, 1 hour, 2 hours and 3 hours after the administration and sera were separated. One week later, 2 g of the same immunoglobulin dissolved in 200 ml of PBS (pH 7.4) was administered orally to the same four volunteers and blood samples were taken according to the same schedule as described above. Sera were also separated from the collected samples. The neutralizing antibody against rotavirus in the separated sera was measured by the plaque reduction procedure using strain NCDV. The anti-rotavirus neutralizing antibody titre in sera was expressed in terms of dilutions.

Table 8

| Neutralizing Antibody Titre in Sera | | | | | | |
|---|---|---|---|---|---|---|
| Volunteer | Dosage form | Neutralizing antibody titre | | | | |
| | | 0 | 30 min. | 60 min. | 120 min. | 180 min. |
| 1 | MFGM emulsion | 1:20 | 1:640 | 1:1280 | 1:1280 | 1:640 |
| | PBS solution | 1:20 | 1:20 | 1:20 | 1:20 | 1:40 |
| 2 | MFGM emulsion | 1:<10 | 1:320 | 1:640 | 1:1280 | 1:640 |
| | PBS solution | 1:<10 | 1:20 | 1:<10 | 1:20 | 1:40 |
| 3 | MFGM emulsion | 1:20 | 1:1280 | 1:640 | 1:1280 | 1:1280 |
| | PBS solution | 1:20 | 1:20 | 1:10 | 1:20 | 1:10 |
| 4 | MFGM emulsion | 1:10 | 1:320 | 1:640 | 1:320 | 1:640 |
| | PBS solution | 1:10 | 1:20 | 1:40 | 1:40 | 1:80 |

As is clear from Table 8, when the immunoglobulin was administered orally as an MFGM emulsion, the neutralizing antibody titre in blood rose significantly compared to the case where it was administered as a PBS solution. It is therefore certain that a substantial amount of immunoglobulin is absorbed through the intestinal tract when it is administered orally as an MFGM emulsion.

As described on the foregoing pages, the method of the present invention which relies upon the use of MFGM and the pharmaceutical composition prepared by this method insure that lipid-soluble drugs and physiologically active peptides administered orally can be absorbed at significantly improved rates and quantities compared to the case where they are formulated in dosage form by the prior art techniques. A further advantage of the present invention lies in its safety since MFGM is a surfactant derived from edible products.

## Claims

1. A method of forming a suspension using fatty globule membranes from mammalian milk as a suspending agent.

2. A method of forming a suspension of a lipid-soluble material that is slightly soluble or insoluble in water and that is soluble in organic solvents, which method is characterized by coating said lipid-soluble material with fatty globule membranes from mammalian milk.

3. A method of forming a fine aqueous suspension of a material that is slightly soluble in both water and an organic solvent but the partition coefficient of which is more favorable to the organic solvent than water, which method is characterized by coating said material with fatty globule membranes from mammalian milk.

4. A method of forming a fine aqueous suspension of a lipid-soluble material that is slightly soluble or insoluble in water and that is soluble in organic solvents, which method is characterized in that fatty globule membranes from mammalian milk and said lipid-soluble material are emulsified in water by mechanical or physical means.

5. A method of forming a fine aqueous suspension of a material that is slightly soluble in both water and an organic solvent but the partition coefficient of which is more favorable to the organic solvent than water, which method is characterized in that fatty globule membranes from mammalian milk and said material are emulsified in water by mechanical or physical means.

6. A method of forming a fine aqueous suspension of a physiologically active peptide by emulsifying said peptide with the aid of fatty globule membranes from mammalian milk.

7. A method of forming a suspension of a physiologically active peptide, which method comprises dissolving or suspending said peptide in water, adding a neutral fat and fatty globule membranes from mammalian milk, and emulsifying the mixture by mechanical or physical means.

8. A method of forming a suspension of a physiologically active peptide, which method comprises dissolving or suspending said peptide in water, adding a neutral fat, fatty globule membranes from

17

mammalian milk and a protease inhibitor, and emulsifying the mixture by mechanical or physical means.

9. A method according to any one of Claims 1 to 8, wherein said fatty globule membranes from mammalian milk are purified fatty globule membranes from cow milk.

10. A method according to any one of Claims 1 to 9, wherein said fatty globule membranes from mammalian milk are replaced by a dairy product containing cow milk fatty globule membranes.

11. A method according to Claim 10 wherein the dairy product containing cow milk fatty globule membranes is buttermilk or cream.

12. A process for producing a pharmaceutical preparation suitable for oral administration, which process is characterized in that a suspended formulation prepared by any one of the methods described in Claims 1 to 11 is either directly, or after addition of an extender, spray-dried or freeze-dried to form a powder.

13. A composition prepared by any one of the methods described in Claims 1 to 12.

14. A pharmaceutical composition prepared by any one of the methods described in Claims 1 to 12.

# Fig. 1

SOLUBILIZING ABILITY

# Fig. 2

## EMULSION STABILITY

Legend:
◆ : LECITHIN
■ : TWEEN 80®
▲ : MFGM
▼ : HCO60

Axes: EMULSION STABILITY VALUE(×100) vs TIME (hrs)

EP 0 385 445 A2

# F I g. 3

EMULSION ACTIVITY
−EFFECT OF SURFACTANT−

● : BUFFER  SOLUTION

◆ : LECITHIN

■ : TWEEN® 80

▼ : HCO 60

▲ : MFGM

# Fig. 4

EMULSION ACTIVITY
—EFFECT OF CONCENTRATION OF MFGM—

# Fig. 5

EMULSION ACTIVITY
—EFFECT OF SURFACTANT—

# Fig. 6

## EMULSION ACTIVITY
### —EFFECT OF LIPID—

# Fig. 7

LYMPH LEVEL OF VITAMIN A AFTER
ADMINISTRATION OF EMULSION IN BILE
FISTULA RAT SMALL INTESTINE

Fig. 8

LYMPH LEVEL OF VITAMIN A AFTER
ADMINISTRATION OF EMULSION IN
INTACT RAT SMALL INTESTINE

Fig. 9

LYMPH LEVEL OF VITAMIN A AFTER
ADMINISTRATION OF EMULSION IN
INTACT RAT SMALL INTESTINE

▲: BUTTEROIL

O: TRIOLEIN

◐: MONOOLEIN

●: OLEIC ACID

# F I g. 10

BLOOD LEVEL OF ³H−HYDROCORTISONE
AFTER ITS ORAL ADMINISTRATION IN RAT

o: EMULSION STABILIZED WITH MFGM

●: SOLUTION STABILIZED WITH TWEEN®80

TIME(hrs.)

# F I g. 11

# F I g. 12